# EUROPÄISCHE PATENTSCHRIFT

(11) **EP 1 284 948 B1**
(45) Veröffentlichungstag und Bekanntmachung des Hinweises auf die Patenterteilung: **09.08.2006**
(21) Anmeldenummer: 01949347.7
(22) Anmeldetag: 17.05.2001
(51) Int. Cl.: C07C 29/141, C07C 31/02

(54) **VERFAHREN ZUR HERSTELLUNG VON GESÄTTIGTEN C 3-C 20-ALKOHOLEN**
METHOD OF PRODUCING SATURATED C 3-C 20 ALCOHOLS
PROCEDE DE PRODUCTION D'ALCOOLS C 3-C 20 SATURES

(30) Priorität: 18.05.2000 DE 10024542
(43) Veröffentlichungstag der Anmeldung: 26.02.2003
(73) Patentinhaber: BASF Aktiengesellschaft, 67056 Ludwigshafen (DE)
(72) Erfinder: HOFFMANN, Herwig, 67227 Frankenthal (DE); RÖPER, Michael, 67157 Wachenheim (DE); BLANKERTZ, Heinrich-Josef, 67147 Forst (DE); STROHMEYER, Max, 67117 Limburgerhof (DE); WALZ, Helmut, 67069 Ludwigshafen (DE); ZINKE-ALLMANG, Helmut, 67098 Bad Dürkheim (DE)
(74) Vertreter: Kinzebach, Werner
(86) Internationale Anmeldenummer: PCT/EP2001/005676
(87) Internationale Veröffentlichungsnummer: WO 2001/087809

(56) Entgegenhaltungen:
- EP-A- 0 004 122
- WO-A-99/31035
- GB-A- 2 097 390

## Beschreibung

Die vorliegende Erfindung betrifft ein Verfahren zur Herstellung von gesättigten C₃-C₂₀-Alkoholen, bei dem man einen flüssigen Hydrierzulauf, der wenigstens einen im Folgenden näher bezeichneten Aldehyd enthält, in Gegenwart eines wasserstoffhaltigen Gases über ein Bett eines Hydrierkatalysators leitet.

Die katalytische Hydrierung von Aldehyden zur Gewinnung von Alkoholen ist ein seit Jahrzehnten ausgeübtes großtechnisches Verfahren, bei dem eine Vielzahl von Katalysatoren, die im Allgemeinen aus Elementen der VI. bis VIII. sowie der I. Nebengruppe des Periodensystems, insbesondere aus den Elementen Chrom, Mangan, Eisen, Kobalt, Nickel und/oder Kupfer, bestehen, eingesetzt werden. Solche Katalysatoren sind z. B. in der DE-A 32 28 881, DE-A 26 28 987 und DE-A 24 45 303 beschrieben. Die nach diesen Verfahren hergestellten Alkohole finden breite Verwendung, z. B. als Lösungsmittel oder als Weichmacheralkohole.

Bei der Hydrierung, insbesondere bei hohen Hydriertemperaturen, laufen neben der erwünschten Hydrierung des Aldehyds zum Alkohol verschiedene unerwünschte Nebenreaktionen, wie Acetalisierungen oder Aldolisierungen, die Tischtschenko-Reaktion oder die Etherbildung, ab. Diese Nebenreaktionen haben eine Erniedrigung der Produktausbeute zur Folge und erfordern einen erhöhten Aufwand bei der Reinigung des Hydrierprodukts, um den betreffenden Alkohol in der gewünschten Reinheit zu erhalten.

Zur Vermeidung derartiger Nebenreaktionen empfiehlt die DE-A 26 28 897, dem Hydrierzulauf Wasser zuzusetzen. Diese Maßnahme weist aber eine Reihe von Nachteilen auf; so erhöht sich z. B. der zur destillativen Reinigung der erhaltenen Alkohole benötigte Energieaufwand beträchtlich.

Eine andere Möglichkeit zur Verringerung der Nebenproduktbildung besteht in der Erhöhung des Wasserstoffdruckes bei der Hydrierung, wodurch sich die Geschwindigkeit der Hydrierreaktion erhöht, während die Reaktionsgeschwindigkeit der vom Wasserstoffdruck unabhängigen Nebenreaktionen gleichbleibt. Insgesamt nimmt damit die Selektivität bezüglich des gewünschten Hydrierprodukts zu.

Eine Erhöhung des Wasserstoffdruckes ist jedoch mit einem hohen apparativen Aufwand verbunden, da aus Sicherheitsgründen Druckreaktoren mit stärkeren Wandungen verwendet und weitere Sicherheitsvorkehrungen getroffen werden müssen.

Die DE-PS 16 43 856 beschreibt die Hydrierung von Aldehyden mittels eines kupfer- und/oder nickelhaltigen Katalysators, dessen Oberfläche durch Behandlung mit Alkalimetallhydroxiden auf einen pH-Wert von 6 bis 10 eingestellt wurde. Diese Druckschrift stellt ausdrücklich auf die Verwendung der so vorbehandelten Katalysatoren bei der Gasphasenhydrierung ab. Ihre Verwendung bei der Flüssigphasenhydrierung ist nur bedingt möglich. Üblicherweise wird das Alkalihydroxid durch den flüssigen Hydrierzulauf bzw. die flüssigen Hydrierungsprodukte ausgewaschen und aus dem Reaktionssystem entfernt, so dass die Vorteile der Oberflächenbehandlung des Katalysators nur kurzfristig sind.

Die JP 61-172 838 A betrifft die Hydrierung von C₅- und höheren Aldehyden an einem Nickel-Chrom-Katalysator in Gegenwart eines tertiären aliphatischen Amins.

Die JP 61-171 447 A betrifft die Hydrierung von C₄-Aldehyden zu Butanol an einem Nickel-Chrom-Katalysator in Gegenwart eines tertiären aliphatischen Amins. Bei den beiden letztgenannten Verfahren wird das zugesetzte Amin durch anschließende Destillation vom Hydrierungsprodukt abgetrennt und zweckmäßigerweise in die Hydrierung zurückgeführt. Bei der Destillation wird allerdings nicht reines Amin zurückgewonnen, sondern es wird ein Gemisch des Amins mit sogenannten Hochsiedern, d. h. den bei der Hydrierung von Aldehyden entstehenden, höher als der Zielalkohol siedenden Nebenprodukten, erhalten. Die Rückführung des Amin/Hochsieder-Gemisches bedingt, dass stets ein Ballast an Hochsiedern durch die Hydrierung und Destillation im Kreis gefahren wird. Da zur Vermeidung von Aufpegelungen immer ein Teil der Hochsieder ausgeschleust werden muß, der der Bildungsrate der Hochsieder entspricht, sind Aminverluste unvermeidbar und stellen eine zusätzliche wirtschaftliche Belastung für das Verfahren dar.

Die WO 96/26173 beschreibt ein Verfahren zur destillativen Reinigung von C₃-C₁₀-Alkoholen, wobei man in Gegenwart eines Alkalihydroxids destilliert. Diese Druckschrift enthält keinen Hinweis darauf, einem flüssigen Hydrierzulauf eine salzartige Base zuzusetzen.

Der vorliegenden Erfindung liegt daher die Aufgabe zugrunde, ein Verfahren zur Herstellung gesättigter Alkohole aus Aldehyden durch Flüssigphasenhydrierung anzugeben, bei dem die Bildung unerwünschter Nebenprodukte, insbesondere bei Hydriertemperaturen von 150°C und mehr, unterdrückt ist und das frei von den Nachteilen der bekannten Hydrierverfahren ist.

Erfindungsgemäß wird diese Aufgabe durch ein Verfahren gelöst, bei dem man einen einen flüssigen Hydrierzulauf, der wenigstens einen unter Propanal, n-Butanal, iso-Butyraldehyd, Hexanal, Ethylhexanal, Ethylhexenal, Nonenal, Nonanal, Decanal, Decenal sowie den Hydroformylierungsprodukten von Trimerpropylen, Tetramerpropylen, Dimerbuten oder Trimerbuten ausgewählten Aldehyd enthält, in Gegenwart eines wasserstoffhaltigen Gases über ein Bett eines Hydrierkatalysators leitet, das dadurch gekennzeichnet ist, dass man dem Hydrierzulauf eine darin homogen lösliche Menge einer salzartigen Base [M⁺]ₙ [An-] zusetzt, worin [M⁺] für ein Alkalimetallion oder das Äquivalent eines Erdalkalimetallions; [Aⁿ⁻] für ein Anion einer Säure mit einem pKₛ-Wert von mehr als 2, und n für die Wertigkeit des Anions steht.

Der Basenzusatz zum Hydrierzulauf bewirkt, daß die eingangs geschilderten Nebenreaktionen selbst bei Hydriertemperaturen von 150°C und mehr weitestgehend zurückgedrängt werden und man auch bei diesen Hydriertemperaturen sehr reine Alkohole erhält.

Die Art der verwendeten salzartigen Base ist im Allgemeinen nicht kritisch, solange die verwendete salzartige Base zumindest in geringer Konzentration im Hydrierzulauf homogen löslich ist und keine unerwünschten Nebenreaktionen mit dem Aldehyd eingeht. Dementsprechend kann eine Vielzahl salzartiger Basen im erfindungsgemäßen Verfahren mit Erfolg eingesetzt werden.

Die erfindungsgemäß eingesetzten Basen sind salzartig, d. h. sie sind aus Kationen und Anionen aufgebaut; sie umfassen wenigstens ein Alkali- oder Erdalkalimetallkation, wie Lithium-, Natrium-, Kalium-, Magnesium- oder Calciumionen, und ein basisches Anion. Die korrespondierende Säure des basischen Anions weist einen pKₛ-Wert von mehr als 2, vorzugsweise mehr als 4, insbesondere mehr als 8, auf. Bei dem zur Charakterisierung der Säurestärke der korrespondierenden Säure herangezogenen pKₛ-Wert handelt es sich um den negativen dekadischen Logarithmus der Dissoziationskonstante der Säure in verdünnter wässriger Lösung. Die pKₛ-Werte zahlreicher Säuren sind tabelliert und finden sich z. B. im CRC Handbook of Chemistry and Physics, 76. Aufl. 1995, CRC Press; organikum, Autorenkollektiv, 16. Aufl. VEB Deutscher Verlag der Wissenschaften 1986, S. 138; Sykes P., Reaktionsmechanismen der Org. Chemie, 8. Aufl. 1982, S. 307.

Geeignete basische Anionen sind Hydroxid (14), Carbonat (10,33), Hydrogencarbonat (6,35), Phosphat (12,35), Amid (35), Hydrid (39) ; Alkoholate, insbesondere C₁-C₄-Alkanolate, wie Methanolat (16), Ethanolat, n- und iso-Propanolat, Butanolat; Phenolat (10); Carboxylate, wie Acetat (4,76) oder Benzoat (4,21); Carbanionen, wie Butyl (50), Cyclopentadienyl oder Phenyl (40). Die Werte in Klammern geben den pKs-Wert der jeweiligen korrespondierenden Säure an. Neben dem Hydridion selbst kommen auch komplexe Hydride in Betracht, die als Addukte des Hydridions aufgefasst werden können und deren Basizität im Wesentlichen auf das Hydridion zurückgeht, z. B. komplexe Hydride wie [BH₄]⁻ oder [BHR₃]⁻ (mit R = C₁-C₄-Alkyl, z. B. s-Butyl.

Im Allgemeinen sind Hydroxid oder Carbonat bevorzugt.

Zweckmäßige salzartige Basen sind vor allem die Alkalimetallhydroxide und/oder -carbonate, wie Lithium-, Kalium- und Natriumcarbonat, sowie Lithium-, Natrium- und Kaliumhydroxid. Im Allgemeinen sind Natrium- und/oder Kaliumhydroxid bevorzugt. Mit besonderem Vorteil können aber auch Natrium- und/oder Kaliumalkoholate, wie das -methanolat oder -ethanolat oder das Alkoholat des Alkohols, der das Hydrierungsprodukt des im Hydrierzulauf enthaltenen Aldehyds ist, eingesetzt werden.

Die salzartigen Basen werden dem Hydrierzulauf im Allgemeinen in einer Menge zugesetzt, die auf Neutralisationsäquivalent-Basis 0,1 bis 2000 Gew.-ppm, vorzugsweise 0,1 bis 1000 Gew.-ppm, insbesondere 0,1 bis 100 Gew.-ppm, besonders bevorzugt von 0,5 bis 50 Gew.-ppm und insbesondere 1 bis 20 Gew.-ppm, bezogen auf den im Hydrierzulauf enthaltenen Aldehyd, Kaliumhydroxid entspricht. Bei einwertigen basischen Anionen wird z. B. eine der angegebenen Menge Kaliumhydroxid entsprechende molare Menge salzartige Base, bei zweiwertigen basischen Anionen die halbe molare Menge verwendet. Es können auch Gemische unterschiedlicher Basen zugegeben werden.

Aufgrund der geringen eingesetzten Konzentrationen der salzartigen Basen sowie des im Allgemeinen geringen Preises dieser Basen ist eine Rückgewinnung nicht erforderlich oder zweckmäßig.

Der flüssige Hydrierzulauf kann aus einem oder mehreren unverdünnten Aldehyden bestehen. Vorzugsweise werden die Aldehyde jedoch als Lösung in einem inerten Verdünnungsmittel eingesetzt. Geeignete inerte Verdünnungsmittel sind beispielsweise Kohlenwasserstoffe, Ether, wie Diethylether, oder Alkohole. Besonders bevorzugt werden Alkohole als Verdünnungsmittel verwendet, insbesondere derjenige Alkohol, der das Hydrierungsprodukt des zu hydrierenden Aldehyds ist. In einer bevorzugten Ausführungsform wird hierzu eine Teilmenge des Hydrierungsproduktes zurückgeführt und mit dem zu hydrierenden Aldehyd vermischt. Falls verwendet, wird das inerte Verdünnungsmittel vorzugsweise in einer Menge von 0,1 bis 100 Gewichtsteilen, insbesondere 1 bis 50 Gewichtsteile und besonders bevorzugt 5 bis 20 Gewichtsteilen, bezogen auf 1 Gewichtsteil eingesetzten Aldehyd, verwendet. Wird die Hydrierung adiabatisch durchgeführt, d. h. unter Abführung der Reaktionswärme durch das Reaktionsprodukt, wird die Menge des verwendeten inerten Verdünnungsmittels zweckmäßigerweise so bemessen, daß der Temperaturgradient über das Bett des körnigen Katalysators 40°C nicht übersteigt. Wird der Hydrierreaktor hingegen isotherm betrieben, kann der Anteil des inerten Verdünnungsmittels im Hydrierzulauf praktisch beliebig gewählt werden.

Der Hydrierzulauf enthält in der Regel Spuren von Wasser z.B. in der Größenordnung von 1 ppm bis 1 Gew.-%, die durch die Edukte in den vorhergegangenen Synthesestufen eingeführt oder durch Kondensationsreaktionen gebildet worden sind. Diese Spuren von Wasser sind unkritisch für das erfindungsgemäße Verfahren. Bei Verwendung anderer salzartiger Basen als Hydroxiden können durch Umprotonierung und/oder Hydrolyse daraus Hydroxidionen gebildet werden, was im Einklang mit der Erfindung steht.

Das erfindungsgemäße Verfahren kann sowohl chargenweise als auch kontinuierlich, z. B. mit Hilfe von Rohrreaktoren oder Reaktorkaskaden, durchgeführt werden. Das Bett des Katalysators ruht im Allgemeinen auf einem geeigneten Halteorgan im Reaktor. Der Hydrierreaktor kann sowohl in der Sumpf- oder in der Rieselfahrweise betrieben werden. Bevorzugt wird das erfindungsgemäße Verfahren in einer Reaktorkaskade, insbesondere einer Kaskade aus zwei bis fünf Reaktoren, ausgeübt.

Die Zugabe der salzartigen Base kann in fester oder gelöster Form erfolgen; sie erfolgt vorzugsweise in Form ihrer Lösung in Wasser oder einem Alkohol, insbesondere dem Alkohol, der das Hydrierungsprodukt des im Hydrierzulauf enthaltenen Aldehyds ist. Z. B. sind 1 bis 40 gewichtsprozentige Lösungen geeignet. Man kann die Base und den Hydrierzulauf getrennt voneinander in den Hydrierreaktor einführen, wobei sich das Gemisch aus Base und Hydrierzulauf in situ im Reaktor bildet. Insbesondere bei der kontinuierlichen Betriebsweise wird jedoch vorzugsweise ein vorgeformtes Gemisch aus Base und Hydrierzulauf in den Reaktor geleitet. Wird, wie in einer bevorzugten Ausführungsform, eine Teilmenge des Hydrierungsproduktes als Verdünnungsmittel vor den Hydrierreaktor zurückgeführt, erfolgt die Dosierung der Base mit Vorteil in den Rückführstrom, bevor dieser mit dem zu hydrierenden Aldehyd vermischt wird. Auf diese Weise vermeidet man lokale Konzentrationsmaxima der Base beim Kontakt mit dem Aldehyd, die zu unerwünschter Aldolisierung führen können. Wird eine Reaktorkaskade benutzt, kann die erforderliche Basenmenge mit dem Hydrierzulauf in den ersten Reaktor der Kaskade geleitet werden; es ist aber auch möglich, die Base separat in jeden einzelnen Reaktor der Kaskade zu dosieren. Vorzugsweise wird die gesamte Basenmenge gemeinsam mit dem Hydrierzulauf in den ersten Reaktor der Kaskade eingeleitet.

Als Hydrierkatalysator werden die üblicherweise zur Hydrierung von Aldehyden zu Alkoholen herangezogenen Katalysatoren verwendet. Die Art des verwendeten Katalysators ist nicht Gegenstand der vorliegenden Erfindung; die vorteilhaften Effekte, die durch das erfindungsgemäße Verfahren erzielt werden, sind von der Art des verwendeten Hydrierkatalysators im Allgemeinen unabhängig. Dementsprechend kann im erfindungsgemäßen Verfahren eine Vielzahl von Hydrierkatalysatoren verwendet werden, beispielsweise metallhaltige Trägerkatalysatoren mit Metallen der I., VII. und/oder VIII. Nebengruppe des Periodensystems als katalytisch aktiven Komponenten, insbesondere Trägerkatalysatoren mit Rhenium, Platin, Palladium, Rhodium und/oder Ruthenium als katalytisch aktiven Komponenten und Trägermaterialien wie Aluminiumoxid, Titandioxid, Siliciumdioxid, Zirkondioxid, Bariumsulfat; oder Fällungskatalysatoren, die mindestens ein Element aus der I., VI., VII. und/oder VIII. Nebengruppe des Periodensystems enthalten, beispielsweise solche Katalysatoren, wie sie in der DE-A 32 28 881, DE-A 26 28 987 und DE-A 24 45 303 beschrieben sind. Die Katalysatoren liegen vorzugsweise in körniger Form vor und weisen im Allgemeinen eine Teilchengröße von 3 bis 10 mm auf. Die Katalysatoren können in einem oder mehreren Betten im Reaktor angeordnet sein. In den verschiedenen Betten eines Reaktors bzw. den verschiedenen Reaktoren einer Reaktorkaskade können unterschiedliche Katalysatoren verwendet werden.

Das wasserstoffhaltige Gas enthält vorzugsweise mehr als 80 mol-% Wasserstoff; es besteht insbesondere im Wesentlichen aus Wasserstoff. Das wasserstoffhaltige Gas kann im Gleich- oder Gegenstrom zum Hydrierzulauf über das Bett des Hydrierkatalysators geführt werden. Es wird vorzugsweise im Gleichstrom geführt. Die Menge des zugeführten wasserstoffhaltigen Gases wird zweckmäßiger Weise so bemessen, dass das 1,0 bis 1,15-fache der stöchiometrisch erforderlichen Wasserstoffmenge zur Verfügung steht.

Die Hydrierung der Alkohole kann unter an sich üblichen Bedingungen erfolgen. Im Allgemeinen werden dabei erhöhte Temperaturen, z. B. von 100 bis 300°C, vorzugsweise von 120 bis 250°C und insbesondere von 130 bis 200°c, sowie Drücke von 1 bis 700 bar, vorzugsweise von 5 bis 300 bar und besonders bevorzugt von 30 bis 50 bar eingestellt. Die Katalysatoren werden im Allgemeinen mit 0,01 bis 2, vorzugsweise mit 0,1 bis 1 und insbesondere mit 0,2 bis 0,5 1 Aldehyd/l Katalysator pro Stunde belastet. Der Zusatz von Wasser zum Hydrierzulauf ist beim erfindungsgemäßen Verfahren möglich, aber nicht erforderlich.

Die Aufarbeitung der Alkohole erfolgt in der Regel destillativ nach an sich bekannten Verfahren.

Bei den zu hydrierenden Aldehyden handelt es sich um Aldehyde, die von besonderer wirtschaftlicher Bedeutung sind, nämlich Propanal, n-Butanal, iso-Butyraldehyd, Hexanal, Ethylhexanal, Ethylhexenal, Nonenal, Nonanal, Decanal, Decenal sowie die Hydroformylierungsprodukte von Trimer- und Tetramerpropylen sowie Dimer- und Trimerbuten.

Das erfindungsgemäße Verfahren weist eine Reihe von Vorteilen auf:

Die bei der Hydrierung von Aldehyden in der Flüssigphase auftretenden Nebenreaktionen der Acetal- und Etherbildung werden stark zurückgedrängt. Der bisher übliche Zusatz von Wasser zum Hydrierzulauf kann reduziert oder gänzlich unterlassen werden. Dadurch kann der Energieaufwand in der nachfolgenden Destillation des Hydrieraustrags deutlich reduziert werden, da das Wasser im Regelfall über Kopf abdestilliert wird. Die Hydriertemperatur kann erhöht werden, ohne dass eine Zunahme von Nebenreaktionen zu befürchten ist. Dadurch kann die Raum-Zeit-Ausbeute erhöht werden; beispielsweise konnte durch eine Erhöhung der Hydriertemperatur von 140 auf 150°C bei der Hydrierung von Butanal die Belastung des Katalysators mit Butanal bei gleicher Butanolausbeute um 25% gesteigert werden. Eine Zunahme der Etherbildung bei der Erhöhung der Hydriertemperatur, wie sie ohne Basenzusatz zu erwarten ist, wurde nicht beobachtet. Durch die Erhöhung der Hydriertemperatur wird die Hydrierwärme auf höherem Temperaturniveau freigesetzt und kann beispielsweise im Wärmeverbund der Hydrieranalge zur Erzeugung von 4-bar-Dampf genutzt werden. Dies führt zu einer beträchtlichen Energieeinsparung.

Die Erfindung wird durch die folgenden Beispiele näher veranschaulicht.

### Beispiele

Die Hydrierung der Aldehyde wurde in einer Reaktorkaskade, bestehend aus einem adiabatisch betriebenen ersten Reaktor eines Inhalts von 1450 l und einem isotherm bei 130°C betriebenen zweiten Reaktor eines Inhalts von 225 l durchgeführt.

Der Hydrierzulauf aus n-Butanal (erhalten durch die Hydroformylierung von Propen) und Rohbutanol wurde den beiden Reaktoren in der Weise zugeführt, dass die Flüssigkeitsbelastung pro m² Reaktorquerschnittsfläche nicht weniger als 20 m³ pro Stunde betrug. Das Rohbutanol mit dem das Butanal im Hydrierzulauf vermischt wurde, war vorher mittels einer Umwälzpumpe aus dem Sumpf des ersten Hydrierreaktors abgezogen worden. Der zweite Reaktor diente als Nachreaktor, um das im ersten Reaktor nur unvollständig umgesetzte Butanol vollends zu hydrieren. Die Zusammensetzung des Hydrieraustrags wurde vor seiner Destillation gaschromatographisch bestimmt.

### Beispiel 1 (nicht erfindungsgemäß)

In der oben beschriebenen Apparatur wurden 2900 kg eines modifizierten Adkins-Katalysators (Lit.: J. Am. Chem. Soc. 51, 2430 (1929); J. Am. Chem. Soc. 54, 4678 (1932)), der im nicht reduzierten Zustand 35 Gew.-% Kupfer, berechnet als Cu, 31 Gew.-% Chrom, berechnet als Cr, 2,0 Gew.-% Barium, berechnet als Ba und 2,5 Gew.-% Mangan, berechnet als Mn, enthielt, wie oben angegeben mit einem Hydrierzulauf belastet, der aus 325 Gew.-Teilen n-Butanal und 5000 Gew.-Teilen im Kreis geführtem Rohbutanol zusammengesetzt war. Der Adkins-Katalysator war vor seiner Verwendung bei 300°C so lange im Wasserstoffstrom reduziert worden, bis kein Wasser mehr gebildet wurde. Der Wasserstoffdruck im Reaktor betrug 40 bar, die Temperatur am Eingang der Katalysatorschüttung betrug 103°C, am Ausgang des ersten Reaktors betrug sie 132°C. In dieser Weise wurde der Reaktor 180 Tage lang betrieben. Das dabei gewonnene Rohbutanol hatte die folgende Zusammensetzung (wasserfrei):

| | |
|---|---|
| n-Butanol | 98,85 Gew.-% |
| Di-n-butylether | 0,11 Gew.-% |
| Butylbutyrat | 0,12 Gew.-% |
| Butyraldehyd-di-n-butylacetal | 0,89 Gew.-% |

Die Carbonylzahl nach DIN 53 173 als Maß für den Restaldehydgehalt wurde zu 0,1 g/g Rohbutanol ermittelt. Die Carbonylzahl ist diejenige Menge Kaliumhydroxyd in mg, die der Chlorwasserstoffmenge äquivalent ist, welche bei der Oximierung von 1 g Substanz aus Hydroxylamoniumchlorid frei wird.

### Beispiel 2 (erfindungsgemäß)

Wie in Beispiel 1 beschrieben wurde ein Hydrierzulauf aus 430 Gew.-Teilen n-Butanal und 5000 Gew.-Teilen Rohbutanol hydriert, wobei dem Hydrierzulauf 3 Gew.-ppm Kaliumhydroxid, bezogen auf das zugeführte n-Butanal, zugesetzt wurden. Die Temperatur am Eingang der Katalysatorschüttung betrug 102°C, am Ausgang betrug sie 137°C. Der Wasserstoffdruck betrug 40 bar. Der Reaktionsaustrag hatte die folgende Zusammensetzung (wasserfrei):

| | |
|---|---|
| Butanal | 0 Gew.-% |
| n-Butanol | 99,68 Gew.-% |
| Di-n-butylether | 0,01 Gew.-% |
| Butylbutyrat | 0,24 Gew.-% |
| Butyraldehyd-di-n-butylacetal | 0,07 Gew.-% |

Die Carbonylzahl, als Maß für den Restaldehydgehalt des Rohbutanols betrug 0,1 mg/g Rohbutanol. Die Carbonylzahl verschlechterte sich auch nach 60 Tagen Betriebsdauer nicht.

### Beispiel 3 (erfindungsgemäß)

In der oben beschriebenen Apparatur wurde ein Katalysator gemäß DE-A 26 28 987, der im nichtreduzierten Zustand 24 Gew.-% Nickel, berechnet als NiO, 8 Gew.-% Kupfer, berechnet als CuO, 2,0 Gew.-% Mangan, berechnet als MnO, auf 66 Gew.-% SiO₂ als Trägermaterial enthielt, in reduzierter Form in den Reaktor gefüllt. Zur Reduktion war der Katalysator bei 200°C solange im Wasserstoffstrom behandelt worden, bis sich kein Wasser mehr bildete.

Am Kopf des Reaktors wurden 500 Gew.-Teile n-Butanal, 15 Gew.-Teile Wasser und 5000 Gew.-Teile im Kreis geführtes Rohbutanol eingeleitet und eine solche Menge einer Lösung von Kaliumhydroxid in n-Butanol diesem Gemisch zudosiert, dass der Zulauf 7 Gew.-ppm Kaliumhydroxid, bezogen auf zugeführtes Butanal, enthielt. Der Druck im Reaktor betrug 38 bar, die Temperatur am Eingang der Katalysatorschüttung betrug 126°C, am Ausgang des ersten Reaktors war sie auf 150°C angestiegen.

Der Hydrieraustrag hatte nach 142 Tagen Betriebsdauer die folgende Zusammensetzung (wasserfrei):

| | |
|---|---|
| Butanal | 0,01 Gew.-% |
| n-Butanol | 99,41 Gew.-% |
| Di-n-butylether | 0,04 Gew.-% |
| Butylbutyrat | 0,02 Gew.-% |
| Butyraldehyd-di-n-butylacetal | 0,53 Gew.-% |

### Beispiel 4 (nicht erfindungsgemäß)

In die oben beschriebenen Apparatur wurden 960 kg eines Katalysators eingebaut, der 19,6 Gew.-% Kupfer und 0,2 Gew.-% Natrium auf einem Träger von Silicagel-Kugeln mit einem Durchmesser von 3-6 mm enthielt. Der Katalysator wurde vorreduziert.

Am Kopf des 1. Reaktors wurde ein Hydrierzulauf, der aus 425 Gew.-Teilen n-Butanal und 5000 Gew.-Teilen im Kreis geführtem Rohbutanol zusammengesetzt war, aufgegeben. Der Wasserstoffdruck im Reaktor betrug 36 bar, die Temperatur am Eingang der Katalysatorschüttung betrug 81°C, am Ausgang des ersten Reaktors betrug sie 115°C. Das gewonnene Rohbutanol hatte die folgende Zusammensetzung (wasserfrei):

| | |
|---|---|
| n-Butanol | 99,44 Gew.-% |
| Di-n-Butylether | 0,01 Gew.-% |
| Butylbutyrat | 0,01 Gew.-% |
| Butyraldehyd-di-n-Butylacetal | 0,44 Gew.-% |

Die Carbonylzahl nach DIN 53 173 als Maß für den Restaldehydgehalt wurde zu 0,15 mg/g Rohbutanol ermittelt.

### Beispiel 5 (erfindungsgemäß)

Wie in Beispiel 4 beschrieben, wurde ein Hydrierzulauf aus 425 Gew.-Teilen n-Butanal ud 5000 Gew.-Teilen Rohbutanol hydriert, wobei dem Hydrierzulauf 32 Gew.-ppm Kaliumhydroxid, bezogen auf das zugeführte n-Butanal, zugesetzt wurden. Die Temperatur am Eingang der Katalysatorschüttung betrug 81°C, am Ausgang betrug sie 111°C. Das gewonnene Rohbutanol hatte die folgende Zusammensetzung (wasserfrei):

| | |
|---|---|
| n-Butanol | 99,81 Gew.-% |
| Di-n-Butylether | 0 Gew.-% |
| Butylbutyrat | 0,04 Gew.-% |
| Butyraldehyd-di-n-Butylacetal | 0,02 Gew.-% |

Die Carbonylzahl, als Maß für den Restaldehydgehalt betrug <0,01 mg/g Rohbutanol.

### Beispiel 6a (nicht erfindungsgemäß)

Wie in Beispiel 4 beschrieben, wurden 985 kg eines vorreduzierten Katalysators eingebaut, der 24,1 Gew.-% Kupfer und 0,27 Gew.-% Natrium auf einem Träger von Silicagel-Kugeln mit einem Durchmesser von 3-6 mm enthielt.

Der Katalysator wurde wie oben angegeben mit einem Hydrierzulauf belastet, der aus 650 Gew.-Teilen n-Butanal und 5000 Gew.-Teilen im Kreis geführtem Rohbutanol zusammengesetzt war.

Der Wasserstoffdruck im Reaktor betrug 36 bar, die maximale Temperatur in der ersten Katalysatorschüttung betrug 126°C, im zweiten Reaktor betrug sie 120°C. In dieser Weise wurde der Reaktor 120 Tage lang betrieben. Das dabei gewonnene Rohbutanol hatte die folgende Zusammensetzung (wasserfrei):

| | |
|---|---|
| n-Butanol | 99,12 Gew.-% |
| Di-n-Butylether | 0,01 Gew.-% |
| Butylbutyrat | 0,02 Gew.-% |
| Butyraldehyd-di-n-Butylacetal | 0,78 Gew.-% |

Die Carbonylzahl nach DIN 53 173 als Maß für den Restaldehydgehalt wurde zu 0,1 g/g Rohbutanol ermittelt.

### Beispiel 6b (erfindungsgemäß)

Die Hydrierapparatur wurde unter identischen Bedingungen wie Versuch 6a weiter betrieben. Dem Hydrierzulauf wurden 10 Gew.-ppm Kaliumhydroxid, bezogen auf das zugeführte Butanal, zugegeben. Bereits 48 h später hatte der Austrag die folgende Zusammensetzung:

| | |
|---|---|
| n-Butanol | 99,82 Gew.-% |
| Di-n-Butylether | 0 Gew.-% |
| Butylbutyrat | 0,05 Gew.-% |
| Butyraldehyd-di-n-Butylacetal | 0,04 Gew.-% |

Die Carbonylzahl als Maß für den Restaldehydgehalt betrug 0,02 mg/g Rohbutanol.

Dieses Beispiel zeigt, dass die vorteilhaften Wirkungen des erfindungsgemäßen Verfahrens auch bei bereits in längerem Gebrauch befindlichen Katalysatoren erzielt werden und sich die Effekte rasch einstellen.

### Beispiel 7

Es wurde ein Hydrierkatalysator nach DE-A 26 28 987 der folgenden Zusammensetzung verwendet:

| | |
|---|---|
| 24 | Gew.-% Nickel, ber. als NiO |
| 8 | Gew.-% Kupfer, ber. als CuO |
| 2,2 | Gew.-% Mangan, ber. als Mn₃O₄ |
| 0,6 | Gew.-% Natrium, ber. als Na₂O |

Rest SiO₂

Die Hydrierung wurde in der oben beschriebenen Apparatur bei den dort angegebenen Bedingungen durchgeführt. Beide Reaktoren wurden mit insgesamt 1600 1 des bei 300°C im Wasserstoffstrom vorreduzierten Katalysators gefüllt.

Der Hydrierzulauf, der aus 360 bis 475 kg Butanalgemisch und 4200 kg Rohbutanol zusammengesetzt war, wurde den beiden Reaktoren in der Weise zugeführt, dass die Flüssigkeitsbelastung pro m² Reaktorquerschnittsfläche bei 30 bis 40m³ lag. Das Rohbutanol war vorher dem Hydrieraustrag des ersten Reaktors zum Zwecke der Rückführung entnommen worden. Die Temperatur über die Schüttung des ersten, adiabatisch betriebenen Reaktors betrug etwa 30°C.

Die Zusammensetzung der Hydrierausträge wurde vor der Destillation gaschromatographisch ermittelt.

Es wurden 4 Versuche gefahren. Bei den Versuchen A und B wurde keine Base zugesetzt (nicht erfindungsgemäß). Bei den erfindungsgemäßen Versuchen C und C wurden jeweils 10 Gew.-ppm Kaliumhydroxid, bezogen auf den Aldehydzulauf, zudosiert. Die Ergebnisse sind in der nachstehenden Tabelle zusammengefasst.

| | A: | B: |
|---|---|---|
| Einsatzstoffe: | 370 kg Butanalgemisch | 430 kg Butanalgemisch |
| | 4200 kg Rohbutanol | 4200 kg Rohbutanol |
| Temperatur am Eingang des 1.Reaktors: | 138°C | 144°C |
| Druck: | 35 bar | 35 bar |

Zusammensetzung des Hydrieraustrags:

| | A: | B: |
|---|---|---|
| Butanale: | 0,006 Gew.-% | 0,007 Gew.-% |
| Butanole: | 99,2 Gew.-% | 99,44 Gew.-% |
| Di-n-butylether: | 0,19 Gew.-% | 0,32 Gew.-% |
| Butylbutyrat: | 0,03 Gew.-% | 0,03 Gew.-% |
| Butyraldehyd-di-butylacetal: | 0,53 Gew.-% | 0,57 Gew.-% |

| | C: | D: |
|---|---|---|
| Einsatzstoffe: | 430 kg Butanalgemisch | 485 Kg Butanalgemisch |
| | 4200 kg Rohbutanol | 4200 kg Rohbutanol |
| Base: | 10 Gew.-ppm KOH | 10 Gew.-ppm KOH |
| Temperatur am Eingang des 1.Reaktors: | 150°C | 150°C |
| Druck: | 35 bar | 35 bar |

Zusammensetzung des Hydrieraustrags:

| | C: | D: |
|---|---|---|
| Butanale: | 0,01 Gew.-% | 0,02 Gew.-% |
| Butanole: | 99,5 Gew.-% | 99,44 Gew.-% |
| Di-n-butylether: | 0,01 Gew.-% | 0,02 Gew.-% |
| Butylbutyrat: | 0,02 Gew.-% | 0,02 Gew.-% |
| Butyraldehyd-di-butylacetal: | 0,46 Gew.-% | 0,50 Gew.-% |

Aus diesen Ergebnissen folgt, dass mit Hilfe des erfindungsgemäßen Basenzusatzes die Hydriertemperatur und demzufolge die Raum-Zeit-Ausbeute erhöht werden kann, ohne dass die Erhöhung der Hydriertemperatur zu einer Zunahme der Etherbildung führt.

## Patentansprüche

1. Verfahren zur Herstellung von gesättigten C₃-C₂₀-Alkoholen, bei dem man einen flüssigen Hydrierzulauf, der wenigstens einen unter Propanal, n-Butanal, iso-Butyraldehyd, Hexanal, Ethylhexanal, Ethylhexenal, Nonenal, Nonanal, Decanal, Decenal sowie den Hydroformylierungsprodukten von Trimerpropylen, Tetramerpropylen, Dimerbuten oder Trimerbuten ausgewählten Aldehyd enthält, in Gegenwart eines wasserstoffhaltigen Gases über ein Bett eines Hydrierkatalysators leitet, **dadurch gekennzeichnet, dass** man dem Hydrierzulauf eine darin homogen lösliche Menge einer salzartigen Base [M⁺]ₙ [Aⁿ⁻] zusetzt, worin [M⁺] für ein Alkalimetallion oder das Äquivalent eines Erdalkalimetallions; [Aⁿ⁻] für ein Anion einer Säure mit einem pKₛ-Wert von mehr als 2, und n für die Wertigkeit des Anions steht.

2. Verfahren nach Anspruch 1, **dadurch gekennzeichnet, dass** man als salzartige Base ein Alkalimetallhydroxid und/oder Alkalimetallcarbonat verwendet.

3. Verfahren nach Anspruch 1 oder 2, **dadurch gekennzeichnet, dass** man dem Hydrierzulauf eine solche Menge salzartiger Base zusetzt, die auf Neutralisationsäquivalent-Basis 0,1 bis 2000 Gew.-ppm, bezogen auf den im Hydrierzulauf enthaltenen Aldehyd, Kaliumhydroxid entspricht.

4. Verfahren nach einem der vorhergehenden Ansprüche, **dadurch gekennzeichnet, dass** der Hydrierzulauf ein inertes Verdünnungsmittel enthält.

5. Verfahren nach Anspruch 4, **dadurch gekennzeichnet, dass** man als inertes Verdünnungsmittel denjenigen Alkohol verwendet, der das Hydrierungsprodukt des im Hydrierzulauf enthaltenen Aldehyds ist.

## Claims

1. A process for the preparation of saturated C₃-C₂₀-alcohols in which a liquid hydrogenation feed comprising at least one aldehyde selected from propanal, n-butanal, isobutyraldehyde, hexanal, ethylhexanal, ethylhexenal, nonenal, nonanal, decanal, decenal and the hydroformylation products of trimeric propylene, tetrameric propylene, dimeric butene or trimeric butene is passed over a bed of a hydrogenation catalyst in the presence of a hydrogen-containing gas, which comprises adding to the hydrogenation feed an amount, homogeneously soluble therein, of a salt-like base [M⁺]ₙ [Aⁿ⁻], in which [M⁺] is an alkali metal ion or the equivalent of an alkali earth metal ion; [Aⁿ⁻] is an anion of an acid having a pKₛ value of greater than 2, and n is the valency of the anion.

2. The process according to claim 1, wherein the salt-like base used is an alkali metal hydroxide and/or an alkali metal carbonate.

3. The process according to claim 1 or 2, wherein an amount of salt-like base which corresponds, on neutralization equivalent basis, to from 0.1 to 2000 ppm by weight, based on the aldehyde present in the hydrogenation feed, of potassium hydroxide is added to the hydrogenation feed.

4. The process according to one of the preceding claims, wherein the hydrogenation feed comprises an inert diluent.

5. The process according to claim 4, wherein the inert diluent used is the alcohol which is the hydrogenation product of the aldehyde present in the hydrogenation feed.

## Revendications

1. Procédé de préparation d'alcools en C₃-C₂₀ saturés, dans lequel on conduit une alimentation d'hydrogénation liquide, qui contient au moins un aldéhyde choisi parmi du propanal, du n-butanal, de l'iso-butyraldéhyde, de l'hexanal, de l'éthylhexanal, de l'éthylhexénal, du nonénal, du nonanal, du décanal, du décénal ainsi que les produits d'hydroformylation de propylène trimère, de propylène tétramère, de butène dimère ou de butène trimère, par-dessus un lit d'un catalyseur d'hydrogénation, en présence d'un gaz contenant de l'hydrogène, **caractérisé en ce qu'**on ajoute à l'alimentation d'hydrogénation une quantité soluble de manière homogène dans celle-ci d'une base saline [M⁺]ₙ [Aⁿ⁻], dans laquelle [M⁺] représente un ion de métal alcalin ou l'équivalent d'un ion de métal alcalino-terreux, [Aⁿ⁻] représente un anion d'un acide ayant une valeur de pKₐ supérieure à 2, et n représente la valence de l'anion.

2. Procédé suivant la revendication 1, **caractérisé en ce que**, comme base saline, on utilise un hydroxyde de métal alcalin et/ou du carbonate de métal alcalin.

3. Procédé suivant la revendication 1 ou 2, **caractérisé en ce qu'**on ajoute à l'alimentation d'hydrogénation une quantité de base saline qui correspond, sur base d'équivalent de neutralisation, à 0,1 jusqu'à 2 000 ppm en poids, par rapport à l'aldéhyde contenu dans l'alimentation d'hydrogénation, d'hydroxyde de potassium.

4. Procédé suivant l'une des revendications précédentes, **caractérisé en ce que** l'alimentation d'hydrogénation contient un diluant inerte.

5. Procédé suivant la revendication 4, **caractérisé en ce que**, comme diluant inerte, on utilise l'alcool qui est le produit d'hydrogénation de l'aldéhyde contenu dans l'alimentation d'hydrogénation.
